# EUROPEAN PATENT SPECIFICATION

(11) **EP 2 974 769 B1**
(45) Date of publication and mention of the grant of the patent: **27.09.2017**
(21) Application number: 15176464.4
(22) Date of filing: 16.03.2010
(51) Int. Cl.: A61N 1/05

(54) **IMPLANTABLE DEVICE AND TISSUE ANCHOR**
IMPLANTIERBARE VORRICHTUNG UND GEWEBEANKER
DISPOSITIF IMPLANTABLE ET ANCRAGE DE TISSU

(30) Priority: 17.03.2009 US 160765 P; 11.09.2009 US 558143
(43) Date of publication of application: 20.01.2016
(62) Divisional of application: 10710501.7
(73) Proprietor: AMS Research Corporation, Minnetonka, MN 55343 (US)
(72) Inventor: LUND, Robert E., Minnetonka, Minnesota 55343 (US); WANG, Guangjian, Minnetonka, Minnesota 55343 (US)
(74) Representative: Vossius & Partner Patentanwälte Rechtsanwälte mbB

(56) References cited:
- US-A1- 2005 021 119
- US-A1- 2006 173 520
- US-A1- 2009 012 592

## Description

### BACKGROUND OF THE INVENTION

Implantable electronic stimulator devices, such as neuromuscular stimulation devices, have been disclosed for use in the treatment of various pelvic conditions, such as urinary incontinence, fecal incontinence and sexual dysfunction. Such devices generally include one or more electrodes that are coupled to a control unit by electrode leads. Electrical signals are applied to the desired pelvic tissue of the patient through the electrode leads in order to treat the condition of the patient. The electrode leads are typically secured to the tissue using an anchor in the form of a helical coil. Exemplary implantable electronic stimulator devices and uses of the devices are disclosed in U.S. Patent Nos. 6,354,991, 6,652,449, 6,712,772 and 6,862,480.

Urinary incontinence in women has been treated by a surgical method involving the placement of a sling to stabilize or support the bladder neck or urethra of the patient. Varieties of sling procedures are described in U.S. Pub. No. 2002-016382 A1. One type of sling procedure is a pubovaginal sling procedure, which is a minimally invasive surgical method involving the placement (e.g. by the use of a Stamey needle or other ligature carrier) of a sling to stabilize or support the bladder neck or urethra. This procedure does not utilize bone anchors. Rather the sling is anchored in the abdominal or rectus fascia.

U.S. Pub. No. 2007-0260288 A1 generally describes a combination of the above devices. One or more electrodes are attached to a mechanical support, such as a sling, that supports a portion of the urethra of the patient. The electrodes are configured to contact tissue of the patient when the mechanical support is implanted in the patient. A control unit drives the electrodes to apply a current to the tissue that treats a pelvic condition of the patient. U.S. Pub. No. 2009-0012592 A1 generally describes an electrode lead with a body having a proximal end and a distal end, two stimulating electrodes and an anchor. The above-described devices utilize anchors to secure components of the devices, such as electrode leads and/or mechanical supports, in tissue of the patient. It is desirable, for example, that such anchors prevent relative movement between the anchor and the tissue in which the anchor in embedded, are easy to install in the tissue, avoid damaging the tissue during the implantation procedure, operate as electrical stimulators, can be temporarily moved relative to the tissue without significant restriction by the anchor during installation, can be removed without significantly damaging the tissue, and/or have other features or benefits recognized by those skilled in the art.

### SUMMARY

The invention is directed to an electrode lead for facilitating electrical stimulation of tissue in a patient in accordance with claim 1. The electrode lead comprises a lead body, an electrode attached to a distal end of the lead body and a tissue anchor. The electrode comprises a plurality of stimulation elements formed of an electrically conductive material, and at least one insulative element formed of an electrically non-conductive material. The stimulation elements and the at least one insulative element are joined together to form a stacked column of alternating stimulation and insulative elements. The electrode lead also comprises a plurality of electrically conductive wires each extending through the lead body and coupled to one of the stimulation elements. The tissue anchor is configured to facilitate anchoring the electrode to tissue of the patient.

This Summary is provided to introduce a selection of concepts in a simplified form that are further described below in the Detailed Description. This Summary is not indented to identify key features or essential features of the claimed subject matter, nor is it intended to be used as an aid in determining the scope of the claimed subject matter. The claimed subject matter is not limited to implementations that solve any or all disadvantages noted in the Background.

### BRIEF DESCRIPTION OF THE DRAWINGS

FIG. 1 is a side plan view of an exemplary electronic stimulator device, in accordance with the embodiments described herein.
FIG. 2 is a schematic illustration of a pelvic treatment apparatus in accordance with embodiments described herein.
FIGS. 3 and 4 are simplified cross-sectional views of a portion of an electrode lead in accordance with embodiments of the invention.
FIGS. 5 and 6 are simplified side cross-sectional views of an anchor in accordance with embodiments described herein.
FIGS. 7-10 are magnified side cross-sectional views of a suture lock in accordance with embodiments described herein.
FIG. 11 is a simplified side view of an electrode lead comprising an anchor in accordance with embodiments described herein.
FIG. 12 is a side cross-sectional view of the electrode lead of FIG. 11 taken generally along line 12-12.
FIG. 13 is a front cross-sectional view of a distal end of an electrode lead installed within a trocar.
FIG. 14 is a simplified front cross-sectional view of a distal end of an electrode lead comprising an anchor in accordance with embodiments described herein.
FIG. 15 is a partial side view of an installation tool in accordance with embodiments described herein.
FIGS. 16-18 are simplified front cross-sectional views of the distal end of an electrode lead illustrating a method of installing the electrode lead within a trocar.
FIG. 19 is a simplified front cross-sectional view of an electrode lead installed within a trocar along with a tool in accordance with embodiments described herein.
FIG. 20 is a side cross-sectional view of FIG. 19 taken generally along line 20-20.
FIGS. 21-23 illustrate method steps of deploying the distal end of the electrode lead of FIGS. 19 and 20 within tissue of a patient, in accordance with embodiments described herein.
FIGS. 24-26 are simplified side views of an electrode lead installation tool in accordance with embodiments described herein.
FIG. 27 is a block diagram of a kit in accordance with embodiments described herein.

### DETAILED DESCRIPTION OF ILLUSTRATIVE EMBODIMENTS

Embodiments are directed to an anchor that facilitates securing implantable devices or components, such as electrode leads, mechanical supports (e.g., meshes, slings), and other devices or components to internal tissue of a patient, and preventing migration of the devices or components from their intended position.

The tissue in which the anchor may be used includes adipose tissue, muscle tissue or any other tissue of the patient. In one embodiment, the tissue is located in the pelvic region of the patient. In some embodiments, the tissue, in which the anchor is to be embedded, is targeted for electrical stimulation or is adjacent a desired stimulation target site. Embodiments of the invention comprise the individual embodiments described below and combinations of two or more of the embodiments described below.

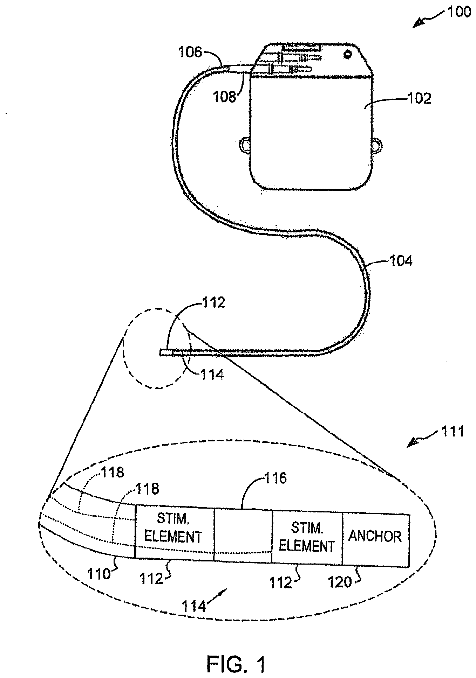

Initially, exemplary devices or components with which the anchors may be used will be discussed. FIG. 1 is a side plan view of an exemplary electronic stimulator device 100, with which embodiments of the anchors may be used. Device 100 is configured for implantation into a pelvic region of a patient to provide muscle and/or nerve stimulation that is used to control and/or treat a pelvic condition of the patient, such as pelvic pain, urinary incontinence, fecal incontinence, erectile dysfunction or other pelvic condition that may be treated through electrical stimulation.

In one embodiment, the device 100 comprises a control unit 102 and one or more electrode leads 104, a proximal end 106 of which is coupled to the control unit 102 via a connector 108. Each electrode lead 104 comprises a lead body 110 and an electrode 111 attached to the lead body 110. One embodiment of the electrode 111 includes one or more stimulation elements 112 at a distal end 114 of the electrode lead 104 or lead body 110. In one embodiment, the stimulation elements 112 are separated from each other by an insulative element 116. The lead body 110 insulates electrical wires 118 connecting the control unit 102 to the stimulation elements 112. The lead body 110 can be in the form of an insulating jacket typically comprising silicone, polyurethane or other flexible, biocompatible electrically insulating material. Additional electrode leads 104 or physiological sensors may be coupled to the control unit 102.

In one embodiment, the control unit 102 comprises circuitry for processing electrical signals received from the one or more stimulation elements 112 or physiological sensors (not shown). The control unit 102 is also configured to apply an electrical current or waveform to the tissue of the patient that is in contact with the one or more stimulation elements 112.

The electrode 111 can be anchored to pelvic tissue of the patient (e.g., internal urinary sphincter muscle) by means of a tissue anchor 120, which is formed in accordance with embodiments described below. The anchor 120 operates to secure the position of the electrode 111 in the desired tissue of the patient. The anchor 120 can be coupled to the lead body 110 or the stimulation element 112. In one embodiment, the anchor 120 operates to provide electrical contact between the pelvic tissue of the patient and the one or more stimulation elements 112 of the electrode 111.

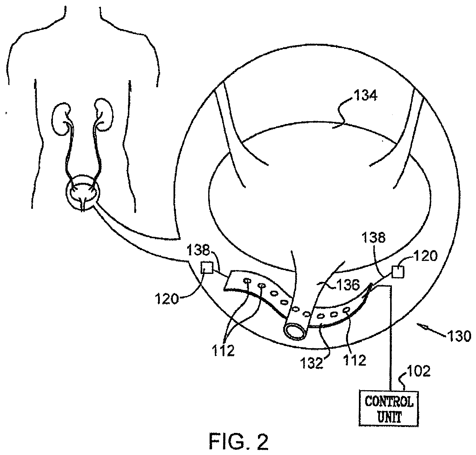

Another device or component with which embodiments of the anchor 120 may be used is a pelvic treatment apparatus 130, an example of which is illustrated in FIG. 2. The pelvic treatment apparatus 130 can be used to treat, for example, urinary incontinence of a patient, and generally comprises a mechanical support 132, which can be in the form of a mesh or other mechanical support. The mechanical support 132 can be installed in the patient to, for example, provide support to the neck of the bladder 134 or the urethra of the patient, which are generally indicated at 136. The mechanical support 132 can be configured for implantation by any number of known surgical approaches, for example, a suprapubic approach, a transvaginal approach, a retropubic approach, and a transobturator approach.

In one embodiment, the mechanical support 132 is anchored to pelvic tissue of the patient using one or more anchors 120 described below. Each anchor 120 can be attached to a cable 138 or directly attached to the mechanical support 132.

In one embodiment, the pelvic treatment apparatus 130 includes one or more stimulation elements 112 that are attached to the mechanical support 132 or extend from the mechanical support 132 on electrode leads (not shown), such as those described above with reference to FIG. 1. A control unit 102, located inside or outside of the patients body, drives the stimulation elements 112 to apply a current to a pelvic site and treat, for example, stress incontinence, urge incontinence, urge frequency, erectile dysfunction, or other pelvic dysfunctions. In accordance with embodiments described herein, the mechanical support 132 and the attached stimulation elements 112 may be referred to as an electrode lead, wherein the support 132 forms the lead body and the stimulation elements 112 form the electrode.

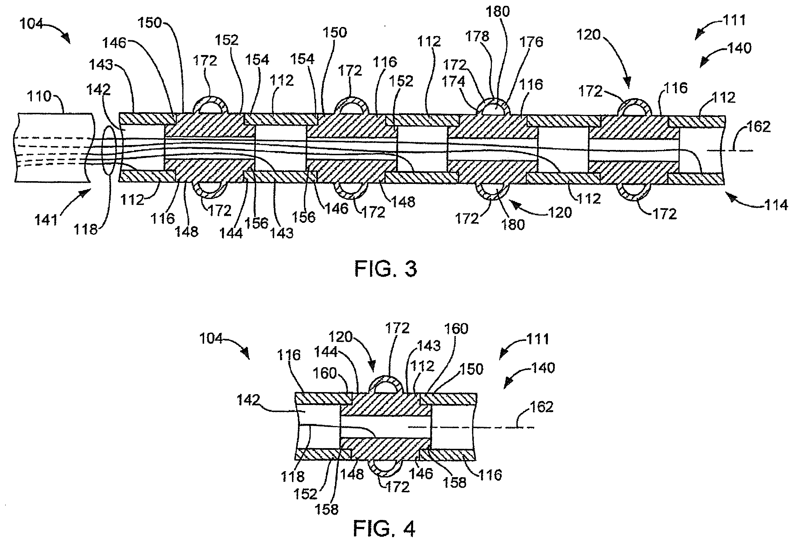

One embodiment of the electrode 111 comprises a stacked column 140 of one or more stimulation elements 112 and insulative elements 116, as illustrated in FIGS. 3 and 4, which are simplified cross-sectional views of a portion of the stacked column 140, in accordance with embodiments of the invention. In one embodiment, the stimulation and insulative elements 112 and 116 are alternated in the stacked column. That is, at least one insulative element 116 is positioned between each pair of stimulation elements 112.

In one embodiment, the stimulation elements 112 are formed of an electrically conductive and biocompatible material, such as alloys of silver, a platinum/iridium alloy (90-10), a nickel-chromium alloy, a gold alloy, or other noble metal alloys. In one embodiment, the stimulation elements 112 are approximately 0.5-5mm in length. The insulative elements 116 are formed of a substantially electrically non-conductive and biocompatible material, such as a suitable polymer.

In one embodiment, electrical wires 118 are coupled to the lead 106 and are configured to deliver electrical signals from the control unit 102 to each of the stimulation elements 112. Each of the wires 118 is electrically coupled to one of the stimulation elements 112, in accordance with known methods. In one embodiment, the wires 118 are fed from a proximal end 141 of the stacked column 140 to the stimulation elements 112 through an internal cavity 142.

In one embodiment, the control unit 102 is configured to selectively apply a desired stimulation waveform to any one of the stimulation elements 112 through the wires 118. This allows for the interrogation of the stimulation elements 112 in order to identify the stimulation element 112 that is producing the best treatment results. Thus, the location of the stimulation can be optimized to produce the desired stimulation effect without having to reposition the electrode 111.

In one embodiment, the stimulation element 112 comprises a body 143, a first end 144 and a second end 146. Similarly, one embodiment of the insulative element 116 comprises a body 148, a first end 150 and a second end 152. In one embodiment, the first end 144 and the second end 146 of the body 143 of the stimulation element 112 are configured to respectively interconnect with the second end 152 and the first end 150 of the body 148 of the insulative elements 116, as shown in FIG. 3. In one embodiment, the first end 144 and/or the second end 146 of the body 143 of the stimulation element 112 comprises a receptacle 154 that is configured to receive a socket or protrusion 156 formed at the first end 150 and/or the second end 152 of the body 148 of the insulative element 116, as shown in FIG. 3. Alternatively, the first end 144 and/or the second end 146 of the body 143 of the stimulation element 112 can include a socket or protrusion 158 that can be received within a receptacle 160 formed in the first end 150 and/or the second end 152 of the body 148 of the insulative element 116, as shown in FIG. 4. The receptacles and protrusions described above operate to facilitate the interconnection of the stimulation elements 112 to the insulative elements 116. A biocompatible adhesive or other conventional means can be used to secure the connections between the stimulation elements 112 and the insulative elements 116, if necessary.

In one embodiment, the stimulation elements 112 and the insulative elements 116 are generally cylindrically shaped and are interconnected such that they are coaxial to a central axis 162.

One embodiment of the electrode lead 104 comprises an anchor 120 (illustrated schematically in FIG. 1) that is configured to facilitate the anchoring of the electrode 111 to the desired tissue of the patient. FIGS. 3 and 4 illustrate an anchor 120 that comprises one or more suture wings 172 that are attached to either the insulative element 116 (FIG. 3) or the stimulation element 112 (FIG. 4). In one embodiment, the suture wings 172 are integral to either the stimulation element 112 or the insulative element 116.

In one embodiment, each suture wing 172 comprises a first end 174, a second end 176 and an intermediary portion 178. The first end 174 and the second end 176 of the suture wing 172 are attached to the body 143 of the stimulation element 112 (FIG. 4), or the body 148 of the insulative element 116 (FIG. 3). The intermediary portion 178 extends between the first end 174 and the second end 176 and is displaced from the corresponding body 143 or 148. The suture wing 172 forms a suture hole 180, through which a suture can be threaded to facilitate the anchoring of the electrode lead 104 to the desired tissue of the patient.

In one embodiment, the width of the electrode 111 including that of the anchor 120 is set to allow the electrode 111 to be deployed into the desired tissue of the patient using a trocar or other device. The width of the anchor 120 can be set based on the location and orientation of the suture wings 172, the displacement of the intermediary portion 178 from the stimulation or insulative elements 112 and 116, and the width of the stimulation and insulative elements 112 and 116. In one embodiment, the fixation element has a width of less than 12mm.

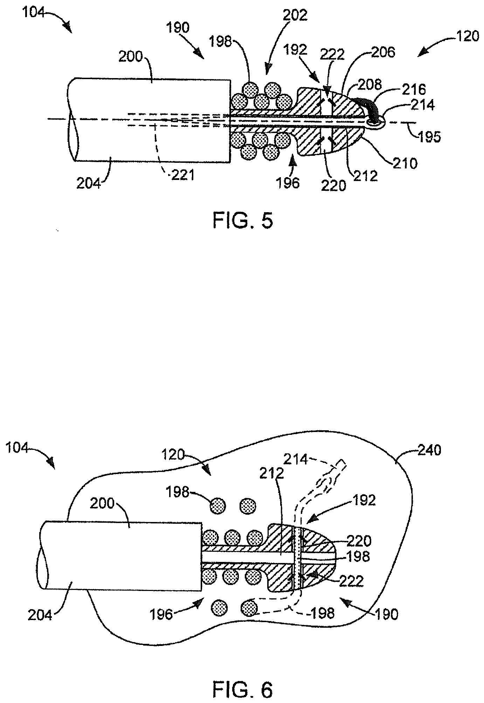

Additional embodiments of the anchor 120 include staggering the suture wings 172 at different radial angles relative to the central axis 162; orienting the suture wings 172 such that they are substantially parallel to the central axis 162 (FIGS. 4 and 5); and orienting the suture wings 172 such that they are at an oblique angle (non-parallel) to the central axis 162; orienting the suture wings 172 such that they are transverse to the central axis 162.

FIGS. 5 and 6 are simplified side-cross sectional views of an anchor 120 that comprises a body 190 and a suture lock 192. The body 190 may be attached to the lead body 110 or the electrode 111 and can be formed of any suitable biocompatible material. In one embodiment, the body 190 is substantially cylindrical in shape in a plane that is transverse to a longitudinal axis 195.

One embodiment of the body 190 comprises a recessed portion 196 that is configured to receive a winding of a suture 198, as shown in FIG. 5. In one embodiment, the recessed portion 196 has a diameter that is sufficiently less than the diameter of a cylindrical portion 200 such that the recessed portion 196 can accommodate a desired amount of the suture 198 without having the suture 198 extend beyond the diameter of the cylindrical portion 200, as shown in FIG. 5. In one embodiment, the recessed portion 196 comprises an annular groove 202 that extends around the longitudinal axis 195 of the body 190. In one exemplary embodiment, the recessed portion 196 can accommodate a length of suture 198 (e.g., Deklene II, Genzyme Corp.) in the range of 3cm; other suitable suture materials may be used.

In one embodiment, the cylindrical portion 200 is positioned at a proximal end 204 of the body 190 and a tapered portion 206 of the body 190 is positioned at a distal end 208. The tapered portion 206 has a diameter that generally decreases with distance along the longitudinal axis 195 of the body 190 in the distal direction, as shown in FIG. 5. The tapered portion 206 can be conical in shape and have a blunt or pointed leading end 210 to facilitate easy insertion into the desired tissue of the patient.

In one embodiment, the body 190 of the anchor 120 includes a needle receptacle 212 that is configured to receive a needle 214, as shown in FIG. 5. In one embodiment, the needle receptacle 212 is formed coaxial to the longitudinal axis 195 and has an opening at the leading end 210. The needle 214 can be removably received within the needle receptacle 212. In one embodiment, the needle 214 can be formed of nickel titanium (NiTi) or other material and has a quiescent shape that is curved. Preferably, the needle 214 can be flexed from the curved quiescent state such that it can be inserted within the needle receptacle 212. In one embodiment, the suture 198 includes an end 216 that is attached to the needle 214 and an opposing end that is attached to the body 190.

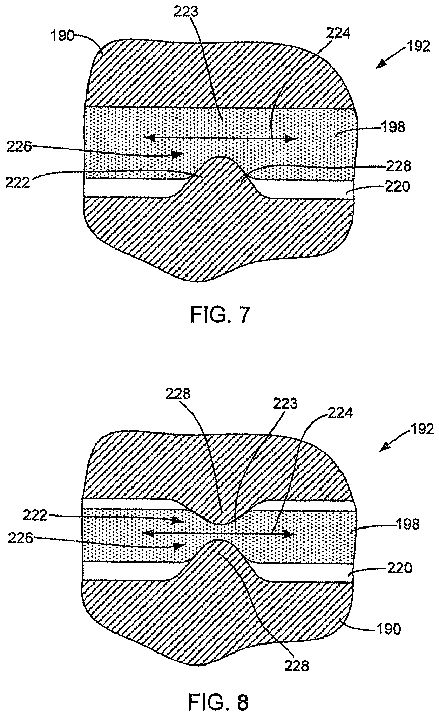

The suture lock 192 is configured to secure an end of the suture 198 to the body 190 of the anchor 120. In one embodiment, the suture lock 192 comprises a passageway 220 that is formed in the body 190 and is configured to receive the suture 198, as shown in FIG. 6. FIGS. 7-10 are magnified side cross-sectional views of the suture lock in accordance with embodiments described herein. One embodiment of the suture lock 192 includes a pinching component 222 that is configured to pinch a portion 223 of the suture 198 and prevent movement of the portion 223 of the suture 198 in a longitudinal direction (represented by arrow 224) relative to the pinching component 222.

In one embodiment, the passageway 220 comprises a bore 221 through the body 190. In one embodiment, the bore of the passageway 220 is transverse to the longitudinal axis 195 of the body 190, as illustrated in FIG. 5. In another embodiment, the passageway 220 passes through the needle receptacle 212, as shown in FIG. 5. In accordance with another embodiment, the passageway 220 is formed on an exterior surface of the body 190. For example, the passageway 220 can be a groove, such as an annular groove, formed on the exterior surface of the body 190.

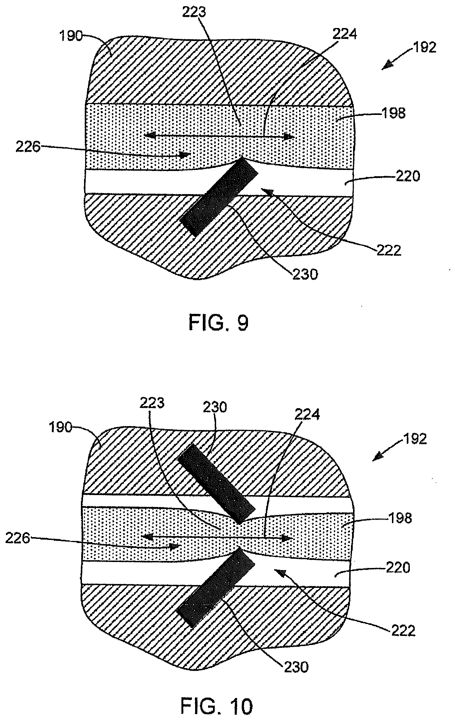

In one embodiment, the pinching component 222 operates to form a radial constriction 226 in the passageway 220 that compresses the suture 198, as illustrated in FIGS. 7-10, and secures the portion 223 of the suture 198 within the passageway 220 to prevent its movement relative to the passageway 220 in the longitudinal direction 224. In one embodiment, the pinching component 222 comprises one or more protuberances 228 formed by the walls of the passageway 220, as illustrated in FIGS. 7 and 8, or an insert placed in the passageway 220. In accordance with another embodiment, the pinching component 222 is formed by one or more fingers 230 that extend into the passageway 220, as shown in FIGS. 9 and 10. The fingers 230 may be insert-molded into the body 190 in accordance with conventional techniques.

The anchor 120 is used to anchor the electrode 111 in place after the electrode 111 has been inserted into a desired pelvic muscle or other tissue of the patient. Any conventional technique may be used to place the electrode 111 in the desired tissue of the patient, such as using a trocar. The processes described below for anchoring the electrode 111 using embodiments of the anchor 120 described above can be performed in any surgically appropriate manner, such as endoscopically.

For the embodiments of the anchor 120 illustrated in FIGS. 3 and 4, the surgeon can attach an end of a suture to one of the suture wings 172 and attach the other end of the suture to a needle. The needle can be passed through the tissue of the patient and through the suture holes 180 of one or more of the suture wings 172. The surgeon can then secure the distal end of the suture to the electrode, such as to one of the suture wings 172, and remove the excess suture and needle to complete the anchoring of the electrode lead 104 to the tissue of the patient.

The suture wings 172 could be used with other fastening methods besides suture. For example, surgical clips or staples could be used. These devices are made of stainless steel or titanium and are bent by a delivery instrument. Endoscopic clip appliers are commonly used during less invasive surgical procedures.

When the anchor 120 is formed in accordance with the embodiments described above with regard to FIGS. 5 and 6, the anchor 120 can initially be inserted into the tissue substantially in the form illustrated in FIG. 5. For example, the anchor 120 can include the suture 198 with one end of the suture 198 attached to the body 190 and the other end 216 of the suture 198 attached to the needle 214. In one embodiment, the suture 198 is wound about a recessed portion 196 and the needle 214 is received within a needle receptacle 212. The electrode 111 can then be inserted into the desired tissue of the patient using a trocar or other technique.

Once inserted into the desired location, the surgeon can remove the needle 214 from the needle receptacle 212 and the anchor 120 can be rotated about the longitudinal axis 195 to unwind the suture 198 from the recessed portion 196. In the event that the fixation component lacks the needle 214, the surgeon can attach a needle to the end 216 of the suture 198 after it has been unwound from the anchor 120. The surgeon can thread the unwound suture through the tissue 240 surrounding the electrode lead 104, as illustrated in FIG. 6. The surgeon can then pass the needle 214 through the suture lock 192 such that the suture 198 is received within the passageway 220 of the suture lock 192 and is pinched by the pinching component 222 to secure the suture 198 within the suture lock 192, as described above. The surgeon can then cut the excess suture 198 and remove the needle and excess suture from the patient. The fixation of the suture 198 within the suture lock 192 completes the anchoring of the electrode lead 104 to the tissue of the patient.

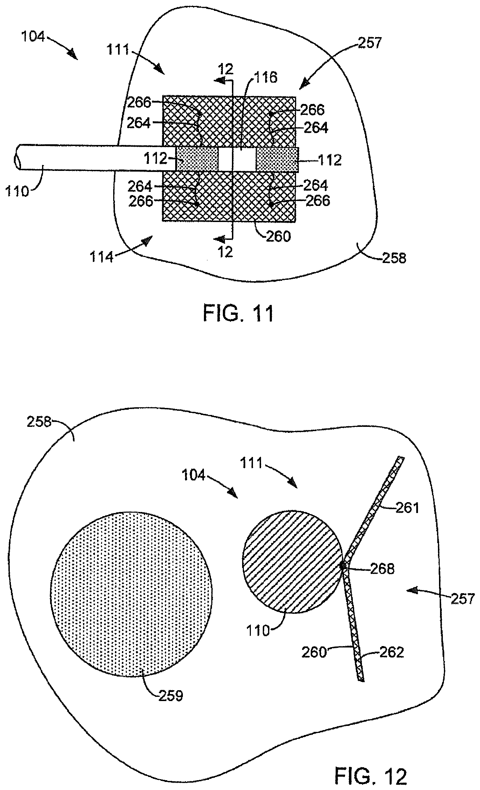

FIG. 11 illustrates a simplified side view of a distal end 114 of an electrode lead 104 having a lead body 110, an electrode 111 and an anchor 257, in accordance with embodiments described herein, that is implanted in tissue 258 of a patient. FIG. 12 provides a cross-sectional view of the anchor 257 taken generally along line 12-12 of FIG. 11 and illustrates the implantation of the distal end 114 of the electrode adjacent a structure 259 of the patient.

As discussed above, one embodiment of the electrode 111 comprises one or more stimulation elements 112, each located at a distal end 114 of the lead body 110. The stimulation elements 112 may be separated by an insulating element 116, which may comprise the lead body 110.

One embodiment of the anchor 257 comprises an anchor body, such as the lead body 110 (FIG. 11) or other component that is attached to the lead body 110, and a section of mesh 260 attached to the anchor body. To simplify the discussion of embodiments of the anchor 257, the anchor body will be described and generally depicted as the lead body 110. The bio-compatible mesh 260 is preferably an open matrix mesh, such as a mesh constructed of polypropylene monofilament. In one embodiment, the mesh 260 comprises one or more mesh sections or wings, such as wings 261 and 262.

In one embodiment, the mesh 260 has a compact state and an expanded state. In general, at least a portion of the mesh 260 is displaced a greater distance from the anchor body 110 when in the expanded state than when in the compact state. The anchor 257 is generally in a form suitable for implantation in the tissue 258 when the mesh 260 is in the compact state and performs its anchoring function in the tissue 258 when the mesh 260 is in the expanded state, in which tissue 258 in-growth through mesh prevents the mesh 260 and the attached stimulation electrodes 112 from moving relative to the tissue 258.

In one embodiment, the mesh 260 has a shape memory that drives the mesh to a preset expanded, quiescent shape, in which at least a portion of the mesh 260 extends away from the anchor body 110 and into the surrounding tissue 258. As used herein, the quiescent shape of the mesh 260 is one in which the mesh will naturally return to after being deformed, such as when compressed into a compact state.

In one embodiment, the expanded state of the mesh wings 261 and 262 is one in which the wings 261 and 262 are displaced from each other, such as illustrated in FIG. 12. Thus, one embodiment of the mesh 260 has a shape memory that encourages separation of the one or more wings, such as wings 261 and 262, within the tissue 258.

In one embodiment, the anchor 257 is configured to deliver electrical signals from the control unit to the tissue 258. In one embodiment, the mesh 260 comprises the one or more stimulation elements 112 of the electrode 111 that are used to deliver electrical signals to the tissue 258. In one embodiment, one or more conductive fibers 264 (FIG. 11) are attached to the mesh 260 and conduct electrical signals from one or more of the stimulation elements 112 of the lead body (FIG. 11), or the control unit 102, into the tissue 258.

In one embodiment, the electrode 111 comprises one or more conductive fibers 264 configured to conduct electrical signals from the control unit 102 to one or more electrically conductive nodes or stimulation elements 266, which are attached to the mesh 260. The wires 264 are electrically insulated from the tissue 258. The stimulation elements 266 deliver the electrical signals to the tissue 258. In one embodiment, the wires 264 electrically couple the stimulation elements 112 to one of the stimulation elements 266.

A portion of the mesh 260 is attached to the distal end 114 of the lead body 110 at a location 268. Exemplary means for attaching the mesh 260 to the lead body 110 include sutures, glue, anchors, or other suitable bio-compatible methods. In one embodiment, the attachment location 268 comprises a central portion of the mesh 260. As a result, one embodiment of the anchor 257 comprises at least two wings of mesh 261 and 262 that extend from the distal end of the lead body 110 at the connection location 268.

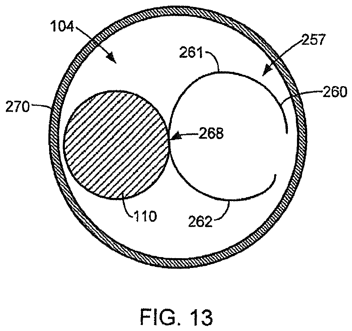

FIG. 13 is a simplified cross-sectional view of the distal end 114 of the lead body 110 having the anchor 257 installed in a delivery trocar 270 that is used to implant the electrode 111 in the desired tissue 258 of the patient. In one embodiment, the mesh 260 of the anchor 257 is placed in the compact state (e.g., rolled up) and installed in the trocar 270. When the anchor 257 is deployed from the trocar 270 into the tissue 258, the mesh 260 expands toward the expanded state or its expanded quiescent shape. Tissue in-growth secures the mesh 260 to the tissue 258 to anchor the location of the electrode 111.

When the mesh 260 comprises the wings 261 and 262, the wings 261 and 262 compressed into the compact state, as shown in FIG. 13. In one embodiment, the mesh wings 261 and 262 extend from the connection point 268 away from the distal end 114 of the lead body 110. Upon deployment of the electrode 111 into the desired tissue 258 of the patient, the mesh sections 261 and 262 move toward the preset expanded quiescent shape and into the tissue 258, as shown in FIG. 12. In-growth of the tissue 258 into the mesh 260 operates to anchor the mesh 260 to the tissue 258, thereby anchoring the electrode 111 in the desired position.

In one embodiment, the mesh 260 is deployed in the tissue 258 such that it has a desired orientation relative to the structure 259 of the patient. As a result, the expansion of the mesh 260 can be directed to a side of the lead body 110 such that it expands away from the structure 259 or toward the structure 259. For instance, when the structure 259 is in the form of the urethra of the patient, the distal end 114 of the lead body 110 can be oriented relative to the urethra 259 such that the mesh 260 is located on a side of the lead body 110 that is away from the urethra 259. This prevents fibrosis around the mesh 260 from interfering with the communication of electrical stimulation signals from the electrode lead 104 to the structure 259.

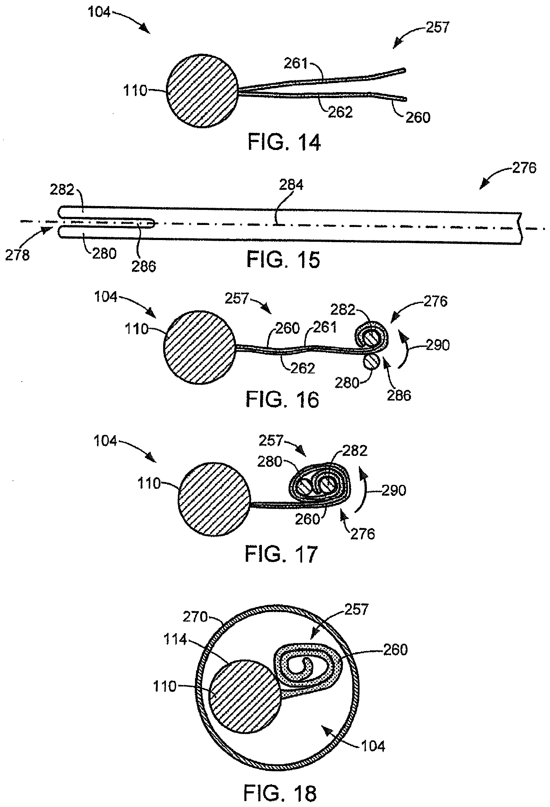

In one embodiment, the anchor 257 is initially provided in a sterilized and sealed package, in which the mesh 260, such as mesh sections 261 and 262, have a quiescent shape in which they lie substantially flat, as illustrated in the simplified cross-sectional view of FIG. 14. FIG. 15 is a simplified side view of an installation tool 276 in accordance with embodiments described herein that is used to prepare the anchor 257 for installation within a trocar 270. The tool 276 comprises a forked end 278 that includes prongs 280 and 282 that extend substantially parallel to the longitudinal axis 284 of the tool 276. A gap 286 between the prongs 280 and 282 is configured to receive an end of the mesh 260.

The anchor 257 is prepared for installation within a trocar 270 by placing an end of the mesh 260 through the gap 286 and rotating the tool 276, such as in the direction indicated by arrow 290, to roll up the mesh 260, as illustrated in simplified front cross-sectional views of FIGS. 16 and 17. Once the mesh 260 has been rolled up into a compact state, the distal end 114 of the lead body 110 and the anchor 257 can be installed in a trocar 270, as illustrated in the simplified cross-sectional view of FIG. 18. The end 278 of the tool 276 is disengaged from the mesh 260 either prior to or after the insertion of the distal end 114 within the trocar 270. When the distal end 114 of the lead body 110 is deployed into the desired tissue 258 of the patient using the trocar 270, the mesh 260 expands toward its expanded quiescent shape within the tissue 258, as discussed above.

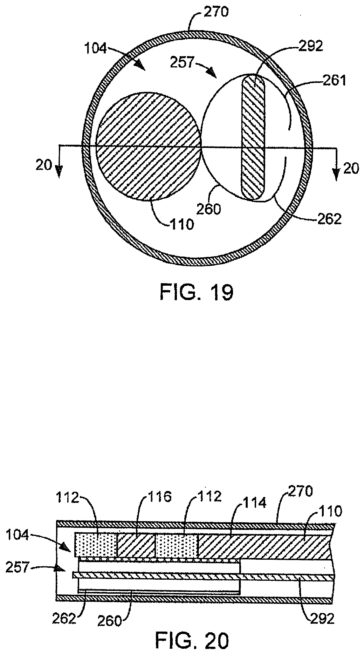

FIGS. 19 and 20 respectively are front and side cross-sectional views of a distal end 114 of the electrode lead 104 comprising the anchor 257 installed within a trocar 270, in accordance with embodiments described herein. A tool 292 is installed in the trocar 270 to aid in the deployment of the mesh wings 261 and 262.

In one embodiment, the tool 292 operates to separate the mesh wings 261 and 262 and prevent their entanglement within the trocar 270.

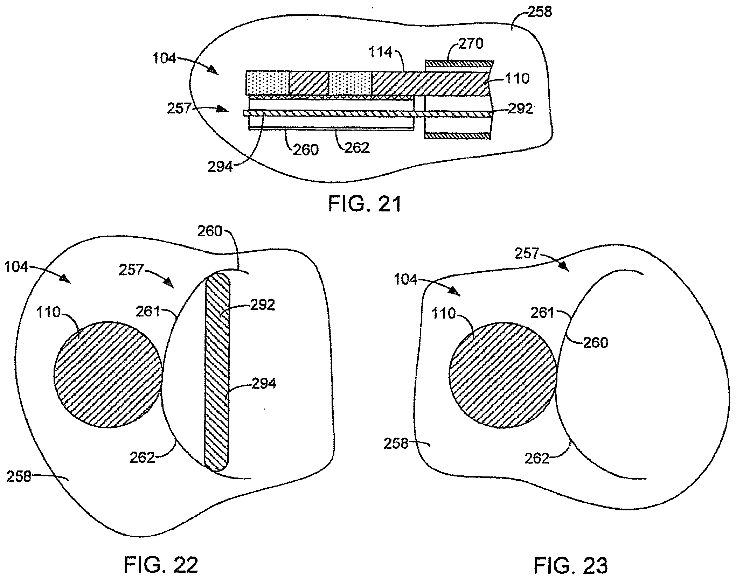

In one embodiment, the tool 292 is configured to cause or assist in the expansion of the mesh wings 261 and 262 into the tissue 258 after deployment of the distal end 114 into the tissue 258. In one embodiment, once the distal end 114 of the lead body 110 held in the trocar 270 is located at the desired position within the tissue 258 of the patient, the trocar 270 is partially retracted, as illustrated in the side cross-sectional view of FIG. 21. In one embodiment, the cross-sectional width of the end 294 of the tool 292 is expanded, which drives the mesh wings 261 and 262 away from each other within the tissue 258, as illustrated in FIG. 22. The distal end 294 of the tool 292 can then be withdrawn into the trocar 270 and removed from the patient through the trocar 270 to complete the implantation of the distal end 114 of the lead body 110 within the tissue 258 of the patient, as illustrated in the cross-sectional view of FIG. 23. In one embodiment, the cross-sectional width of the end 294 of the tool 292 is reduced prior to its withdrawal from the patient.

In one embodiment, the tool 292 comprises a scissor-like mechanism that expands the effective width of the distal end 294.

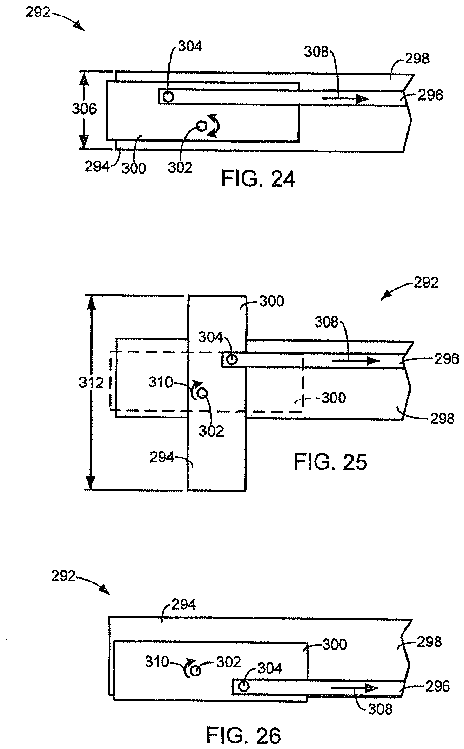

FIGS. 24-26 are simplified side views of a tool 292 in accordance with embodiments described herein. In one embodiment, the tool 292 comprises first and second elongated components 296 and 298 that are respectively coupled to a third component 300 through hinges 302 and 304. The initial width 306 of the tool 292 allows the tool 292 to be received within the trocar 270, as shown in FIGS. 19 and 20. Following the retraction of the trocar 270 (FIG. 21), component 296 is moved relative to component 298, such as, for example, in the direction indicated by arrow 308. The displacement of the hinge 304 from the hinge 302 in the widthwise direction causes the component 300 to pivot about the hinge 302 relative to the component 298 in the direction indicated by arrow 310 to an expanded position, as shown in FIG. 25. The width 312 of the distal end 294 of the tool 292 in the expanded position is significantly greater than the width 306. This expansion of the width of the distal end 294 drives the expansion of the mesh wings 261 and 262, as illustrated in FIG. 22.

After the mesh wings 261 and 262 have been expanded through the expansion of the width of the distal end 294 of the tool 292, the component 296 can be further moved relative to the component 298 in a direction indicated by arrow 308 to place the distal end 294 in a compacted state (FIG. 26), in which the tool 292 may either be received again within the trocar 270, or otherwise removed from the patient, while leaving the distal end 114 of the lead body 110 in the desired location within the tissue 258 of the patient.

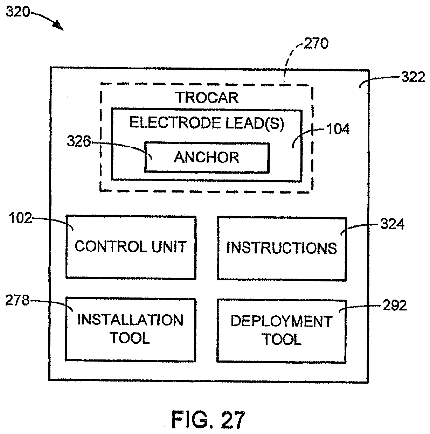

Additional embodiments are directed to kits that include one or more of the embodiments described above. FIG. 27 illustrates a kit 320 in accordance with embodiments described herein. In one embodiment, the kit includes a package 322 containing components including one or more electrode leads 104 in accordance with one or more of the embodiments described above. In one embodiment, the kit 320 includes the control unit 102. In one embodiment, the kit includes instructions 324 for installing the one or more electrode leads 104 in the patient. In one embodiment, the instructions 324 include instructions for installing the control unit 102. In one embodiment, the kit 320 includes the installation tool 278. In one embodiment, the kit 320 includes the deployment tool 292.

In one embodiment, the one or more electrode leads 104 in the kit 320 include an anchor 326 in accordance with one or more of the embodiments described above, such as anchor 120 or 257. In accordance with one embodiment, the distal end 114 of the one or more electrode leads are provided pre-installed in a trocar 270. In accordance with one embodiment, one or more tools, such as installation tool 278 or deployment tool 292, are provided in the kit 320. Embodiments of the instructions 324 include instructions for implanting the distal end 114 of the electrode lead 104 within tissue 258 of the patient using the trocar 270, tool 278 and/or tool 292. Such instructions include instructions describing one or more of the method steps discussed above with reference to FIGS. 16-26.

For example, while some embodiments of the tissue anchor (120,257) described above are specifically described as being used to secure a distal end of an electrode lead to tissue of a patient, those skilled in the art understand that these embodiments of the tissue anchor may also be used to anchor one or more ends of a mechanical support (132).

The application is further characterized by the following items:
1. An implantable electrode lead (104) for facilitating electrical stimulation of tissue (240) of a patient, the electrode lead comprising:
   a lead body (110) having a distal end (114);
   an electrode (111) attached to the distal end of the lead body, the electrode comprising:
      a plurality of stimulation elements (112) formed of an electrically conductive material; and
      at least one insulative element (116) formed of an electrically non-conductive material;
      wherein the stimulation elements and the at least one insulative element are joined together to form a stacked column (140) of alternating stimulation and insulative elements;
   a plurality of electrically conductive wires (118) extending through the lead body, each wire coupled to one of the stimulation elements; and
   a tissue anchor (120, 257) configured to facilitate anchoring the electrode to tissue of the patient.
2. The electrode lead of item 1, wherein the tissue anchor comprises a plurality of suture wings (172), each suture wing comprising first and second ends (174, 176) attached to one of the stimulation elements or one of the at least one insulative element and an intermediary portion (178) extending between the first and second ends, wherein the suture wings each form a suture hole (180), through which a suture can be fed for anchoring the electrode to tissue of the patient.
3. The electrode lead of item 1, wherein the tissue anchor comprises:
   a body (190); and
   a suture lock (192) comprising:
      a passageway (220) formed in the body and configured to receive a portion (223) of a suture (198); and
      a pinching component (222) configured to pinch the portion of the suture within the passageway and prevent movement of the portion of the suture relative to the pinching component.
4. The electrode lead of item 1, wherein:
   each of the stimulation elements comprises a body (143) having first and second ends (144, 146); and
   each of the at least one insulative elements comprises a body (148) having first and second ends (150, 152) that are respectively configured to interconnect with the second and first ends of the body of one of the stimulation elements.
5. The electrode lead of item 4, wherein:
   the second end of the body of the stimulation element comprises a receptacle (154); and
   the first end of the body of the insulative element comprises a protrusion (156) that is received within the receptacle.
6. The electrode lead of item 4, wherein:
   the first end of the body of the insulative element comprises a receptacle (160); and
   the second end of the body of the stimulation element comprises a protrusion (158) that is received within the receptacle.
7. The electrode lead of item 4, wherein the body of the stimulation element and the body of the insulative element are coaxial.
8. The electrode lead of item 1, wherein the tissue anchor comprises mesh (160).
9. The electrode lead of item 8, further comprising one or more electrically conductive wires (264) each coupled to one of the stimulation elements and the mesh.
10. An implantable electrode lead (104) for facilitating electrical stimulation of tissue (240) of a patient, the electrode lead comprising:
   a lead body (110) having a distal end (114);
   an electrode (111) attached to the distal end of the lead body, the electrode comprising at least one stimulation element (112) formed of an electrically conductive material;
   an electrically conductive wire (118) extending through the lead body and coupled to the stimulation element; and
   a tissue anchor (120) attached to the lead body and configured to facilitate anchoring the electrode to tissue of the patient, the tissue anchor comprising:
      an anchor body (190) having a needle receptacle (212);
      a needle (214) removably received within the needle receptacle; and
      a suture (198) having a first end (216) attached to the needle.
11. The electrode lead of item 10, wherein the tissue anchor comprises a suture lock (192) comprising:
   a suture passageway (220) formed in the anchor body and configured to receive a portion (223) of the suture; and
   a pinching component (222) within the suture passageway configured to pinch a portion of the suture within the passageway and prevent movement of the portion of the suture relative to the pinching component.
12. The electrode lead of item 10, wherein the anchor body comprises the at least one stimulation element.
13. The electrode lead of item 10, wherein the needle receptacle is aligned with a longitudinal axis (195) of the body.
14. The electrode lead of item 10, wherein the passageway comprises a bore (221) through the anchor body.
15. The electrode lead of item 10, wherein the pinching component comprises a constriction (226) formed in the passageway.
16. A kit (320) comprising comprising:
   a trocar (270); and
   an electrode lead (104), the electrode comprising:
      a lead body (110) having a distal end (114);
      an electrode (111) formed of an electrically conductive material, the electrode attached to the distal end of the lead body; and
      a tissue anchor (257) attached to the distal end of the lead body, the anchor comprising mesh.
17. The kit of item 16, wherein the distal end of the electrode lead is installed within the trocar.
18. The kit of item 17, further comprising a deployment tool (292) having an expandable distal end (294) positioned within the trocar adjacent the tissue anchor, wherein the expandable distal end is configured to expand the mesh within the tissue of the patient.
19. The kit of item 16, further comprising an installation tool (276) configured to prepare the anchor for installation in the trocar, the installation tool comprising a forked end (278) that includes a pair of prongs (280, 282) separated by a gap (286).
20. The kit of item 16, further comprising a deployment tool (292) having an expandable distal end (294).
21. An implantable device (104, 130) comprising:
   a lead body (110, 132);
   a tissue anchor (120) attached to the lead body comprising:
      an anchor body (190) having a needle receptacle (212);
      a needle (214) removably received within the needle receptacle; and
      a suture (198) having a first end (216) attached to the needle.
22. The device of item 21, wherein the tissue anchor comprises a suture lock (192) comprising:
   a suture passageway (220) formed in the anchor body and configured to receive a portion (223) of the suture; and
   a pinching component (222) within the suture passageway configured to pinch a portion of the suture within the passageway and prevent movement of the portion of the suture relative to the pinching component.
23. The device of item 21, wherein the needle receptacle is aligned with a longitudinal axis (195) of the body.
24. The device of item 21, wherein the pinching component comprises a constriction (226) formed in the passageway.

## Claims

1. An implantable electrode lead (104) for facilitating electrical stimulation of tissue (240) of a patient, the electrode lead comprising:
a lead body (110) having a distal end (114);
an electrode (111) attached to the distal end of the lead body, the electrode comprising:
a plurality of stimulation elements (112) formed of an electrically conductive material; and
at least one insulative element (116) formed of an electrically non-conductive material;
a plurality of electrically conductive wires (118) extending through the lead body, each wire coupled to one of the stimulation elements; and
a tissue anchor (120, 257) configured to facilitate anchoring the electrode to tissue of the patient,
**characterized in that**:
the stimulation elements and the at least one insulative element are joined together to form a stacked column (140) of alternating stimulation and insulative elements;
each of the stimulation elements comprises a body (143) having first and second ends (144, 146); and
each of the at least one insulative elements comprises a body (148) having first and second ends (150, 152) that are respectively configured to interconnect, respectively, with the second end of the body of one of the stimulation elements and with the first end of the body of another one of the stimulation elements.

2. The electrode lead of claim 1, wherein:
the second end of the body of each stimulation element comprises a receptacle (154); and
the first end of the body of each insulative element comprises a protrusion (156) that is received within the receptacle of the second end of the body of the stimulation element interconnected with it.

3. The electrode lead of claim 1, wherein:
the first end of the body of each insulative element comprises a receptacle (160); and
the second end of the body of each stimulation element comprises a protrusion (158) that is received within the receptacle of the first end of the body insulative element interconnected with it.

4. The electrode lead of claim 1, wherein the body of the stimulation element and the body of the insulative element are coaxial.

## Patentansprüche

1. Implantierbare Elektrodenleitung (104) zur Erleichterung von elektrischer Stimulation von Gewebe (240) eines Patienten, wobei die Elektrodenleitung aufweist:
einen Leitungskörper (110) mit einem distalen Ende (114);
eine Elektrode (111), die am distalen Ende des Leitungskörpers angebracht ist, wobei die Elektrode aufweist:
mehrere Stimulationselemente (112), die aus einem elektrisch leitenden Material gebildet sind; und
mindestens ein Isolierelement (116), das aus einem elektrisch nicht leitenden Material gebildet ist;
mehrere elektrisch leitende Drähte (118), die sich durch den Leitungskörper erstrecken, wobei jeder Draht mit einem der Stimulationselemente gekoppelt ist; und
einen Gewebeanker (120, 257), der so konfiguriert ist, dass er das Verankern der Elektrode an Gewebe des Patienten erleichtert,
**dadurch gekennzeichnet dass**:
die Stimulationselemente und das mindestens eine Isolierelement so zusammengefügt sind, dass sie eine Stapelsäule (140) aus abwechselnden Stimulations- und Isolierelementen bilden;
wobei jedes Stimulationselement einen Körper (143) mit einem ersten und einem zweiten Ende (144, 146) aufweist; und
jedes des mindestens einen Isolierelements einen Körper (148) mit einem ersten und einem zweiten Ende (150, 152) aufweist, die jeweils so konfiguriert sind, dass sie mit dem zweiten Ende des Körpers eines der Stimulationselemente bzw. mit dem ersten Ende des Körpers eines anderen der Stimulationselemente gegenseitig verbunden sind.

2. Elektrodenleitung nach Anspruch 1, wobei
das zweite Ende des Körpers von jedem Stimulationselement eine Aufnahme (154) aufweist; und
das erste Ende des Körpers jedes Isolierelements einen Vorsprung (156) aufweist, der in der Aufnahme des zweiten Endes des Körpers des mit ihm verbundenen Stimulationselements aufgenommen ist.

3. Elektrodenleitung nach Anspruch 1, wobei
das erste Ende des Körpers jedes Isolierelements eine Aufnahme (160) aufweist; und
das zweite Ende des Körpers jedes Stimulationselements einen Vorsprung (158) aufweist, der in der Aufnahme des ersten Endes des Körpers des mit ihm verbundenen Isolierelements aufgenommen ist.

4. Elektrodenleitung nach Anspruch 1, wobei der Körper des Stimulationselements und der Körper des Isolierelements koaxial sind.

## Revendications

1. Fil d'électrode implantable (104) permettant de faciliter la stimulation électrique d'un tissu (240) d'un patient, le fil d'électrode comprenant :
un corps de fil (110) ayant une extrémité distale (114) ;
une électrode (111) fixée à l'extrémité distale du corps de fil, l'électrode comprenant :
une pluralité d'éléments de stimulation (112) formés d'un matériau électriquement conducteur ; et
au moins un élément isolant (116) formé d'un matériau électriquement non-conducteur ;
une pluralité de fils électriques (118) électriquement conducteurs s'étendant à travers le corps de fil, chaque fil électrique étant couplé à l'un des éléments de stimulation ; et
une ancre de tissu (120, 257) configurée pour faciliter l'ancrage de l'électrode au tissu du patient ;
**caractérisé en ce que**
les éléments de stimulation et l'au moins un élément isolant se joignent ensemble pour former une colonne empilée (140) d'éléments de stimulation et d'isolation alternant ;
chacun des éléments de stimulation comprenant un corps (143) ayant une première et une seconde extrémité (144, 146) ; et
chacun des au moins un élément isolant comprenant un corps (148) ayant une première et une seconde extrémité (150, 152), qui sont respectivement configurées pour s'interconnecter, respectivement, avec la seconde extrémité du corps de l'un des éléments de stimulation et avec la première extrémité du corps d'un autre des éléments de stimulation.

2. Fil d'électrode selon la revendication 1, dans lequel la seconde extrémité du corps de chaque élément de stimulation comprend un réceptacle (154); et la première extrémité du corps de chaque élément isolant comprend une saillie (156) qui est reçu à l'intérieur du réceptacle de la seconde extrémité du corps de l'élément de stimulation interconnecté avec celui-ci.

3. Fil d'électrode selon la revendication 1, dans lequel la première extrémité du corps de chaque élément isolant comprend un réceptacle (160); et la seconde extrémité du corps de chaque élément de stimulation comprend une saillie (158) qui est reçu à l'intérieur du réceptacle de la première extrémité du corps de l'élément isolant interconnecté avec celui-ci.

4. Fil d'électrode selon la revendication 1, dans lequel le corps de l'élément de stimulation et le corps de l'élément isolant sont coaxiaux.
